# EUROPEAN PATENT APPLICATION

(11) **EP 3 682 804 A1**
(43) Date of publication of application: **22.07.2020**
(21) Application number: 20151704.2
(22) Date of filing: 14.01.2020
(51) Int. Cl.: A61B 6/00, A61B 6/02, G06T 7/00, G06T 11/60

(54) **X-RAY IMAGING APPARATUS AND CONTROL METHOD THEREOF**

(30) Priority: 18.01.2019 KR 20190006554; 06.01.2020 KR 20200001665
(71) Applicant: Samsung Electronics Co., Ltd., Gyeonggi-do 16677 (KR)
(72) Inventor: JUNG, Ji Young, 16677 Gyeonggi-do (KR)
(74) Representative: Hylarides, Paul Jacques

(57) **Abstract**

The disclosure provides an X-ray imaging apparatus and control method thereof, which allows the user to diagnose a disease more quickly by determining a sectional image including a feature corresponding to a disease among a plurality of sectional images obtained by reconstructing a plurality of projected images corresponding to a plurality of locations, and displaying an indicator to indicate the feature or a numerical value that scores the disease in the sectional image. According to an embodiment of the disclosure, an X-ray imaging apparatus includes an X-ray source irradiating X-rays to a subject from a plurality of locations; an X-ray detector detecting X-rays irradiated from the plurality of locations and passing through the subject; and a controller configured to obtain a plurality of projected images corresponding to the plurality of locations from the detected X-rays, create a plurality of sectional images by reconstructing the plurality of projected images, determine at least one of the plurality of sectional images having a preset feature in a region of interest, and insert an indicator to indicate the feature to the at least one sectional image.

## Description

### BACKGROUND

### 1. Field

The disclosure relates to an X-ray imaging apparatus and control method thereof, which images an internal portion of a subject.

### 2. Discussion of Related Art

X-ray imaging apparatuses are devices for allowing the user to see an internal structure of a subject by irradiating X-rays to the subject and analyzing X-rays that have passed through the subject.

In general, the X-ray imaging apparatus uses a single fixed X-ray source to scan a subject from a single view point and image the internal structure of the subject in a two dimensional image, thus having a limitation in distinguishing normal tissues from diseases.

An X-ray imaging apparatus based on a tomosynthesis system has recently been released, which scans a subject from different angles or different view points by moving an X-ray source or using a plurality of X-ray sources arranged at different positions, thereby providing a plurality of tomographic images and allowing the user to check the normal tissues and diseases more closely.

### SUMMARY

One or more example embodiments provide an x-ray imaging apparatus and control method thereof, which allows the user to diagnose a disease more quickly by determining a sectional image including a feature corresponding to a disease among a plurality of sectional images obtained by reconstructing a plurality of projected images corresponding to a plurality of locations, and displaying an indicator to indicate the feature or a numerical value that scores the disease in the sectional image.

According to an embodiment of the disclosure, an X-ray imaging apparatus includes an X-ray source irradiating X-rays to a subject from a plurality of locations; an X-ray detector detecting X-rays irradiated from the plurality of locations and passing through the subject; and a controller configured to obtain a plurality of projected images corresponding to the plurality of locations from the detected X-rays, create a plurality of sectional images by reconstructing the plurality of projected images, determine at least one of the plurality of sectional images having a preset feature in a region of interest, and insert an indicator to indicate the feature to the at least one sectional image.

The X-ray imaging apparatus may further include a display, and the controller may control the display to display the at least one sectional image to which the indicator is inserted.

The preset feature may include at least one type of a foreign body in a joint, a joint cartilage defect, bone fractures, a joint having maximum thickness, or a joint having minimum thickness.

The controller may perform image processing on the region of interest of each of the plurality of sectional images to determine whether there is the preset feature in each of the plurality of sectional images.

The controller may perform image processing on the region of interest in each of the plurality of sectional images by separating a joint image and a bone image included in the region of interest in each of the plurality of sectional images.

The controller may determine at least one of whether there is a foreign body in the joint, whether there is a joint cartilage defect, or whether there is bone fracture in each of the plurality of sectional images based on image processing on the joint image and bone image in each of the plurality of sectional images.

The controller may measure joint thickness in the region of interest in each of the plurality of sectional images based on the joint image and the bone image in each of the plurality of sectional images.

The controller may determine at least one of a sectional image having maximum joint thickness or a sectional image having minimum joint thickness among the plurality of sectional images, and insert an indicator to indicate a joint to the determined sectional image.

The controller may perform a neural network operation on each of the plurality of sectional images, and determine whether there is the preset feature in each of the plurality of sectional images based on information resulting from the neural network operation for the sectional image.

The controller may control the display to match and display each type of the preset feature and an identification number of a sectional image corresponding to the type of the preset feature.

The X-ray imaging apparatus may further include an input device configured to receive an input of a user, and the controller may control the display to display one of the plurality of sectional images, receive a choice of at least one region of interest including a portion of interest in the sectional image from the user through the input device.

When a preset input is received from the user through the input device, the controller may control the display to display the at least one of the plurality of sectional images to which the indicator is inserted.

The controller may perform image processing on one of the plurality of sectional images to determine a joint included in the sectional image, and determine a region of interest to include the determined joint.

When there exist a plurality of previous sectional images of the subject including the region of interest and previously obtained, the controller may control the display to display at least one previous sectional image with an indicator inserted to the region of interest among the plurality of previous sectional images and at least one sectional image to which the indicator is inserted.

The controller may determine whether there is a change in the feature by comparing the at least one sectional image with the at least one previous sectional image, and control the display to display information about the determination of whether there is a change in the feature.

According to an embodiment of the disclosure, an X-ray imaging apparatus includes an X-ray source irradiating X-rays to a subject from a plurality of locations; an X-ray detector detecting X-rays irradiated from the plurality of locations and passing through the subject; and a controller configured to obtain a plurality of projected images corresponding to the plurality of locations from the detected X-rays, create a plurality of sectional images by reconstructing the plurality of projected images, determine at least one of the plurality of sectional images having a preset feature in a region of interest, and determine a numerical value corresponding to the region of interest based on a state of the feature in the at least one sectional image.

The controller may calculate a numerical value for the region of interest in each of the at least one sectional image based on information about correlations between features and numerical values and respective feature in the at least one sectional image, and determine a numerical value corresponding to the region of interest by summing the calculated numerical values.

The controller may perform a neural network operation on each of the at least one sectional image, calculate a numerical value for the region of interest of each of the at least one sectional image based on information resulting from the neural network operation for the sectional image, and determine a numerical value corresponding to the region of interest by summing the calculated numerical values.

The X-ray imaging apparatus may further include a display and the controller may control the display to display at least one of the numerical values for the respective regions of interest in one of the plurality of sectional images or a sum of the numerical values.

The X-ray imaging apparatus may further include an input device receiving an input of a user, and the controller may, when receiving a choice of one of regions of interest from the user through the input device, control the display to display at least one of a sectional image having the feature corresponding to the chosen region of interest or a calculation ground for the numerical value for the chosen region of interest.

According to an embodiment of the disclosure, a control method of an X-ray imaging apparatus including an X-ray source irradiating X-rays to a subject from a plurality of locations and an X-ray detector detecting X-rays irradiated from the plurality of locations and passing through the subject, includes: obtaining a plurality of projected images corresponding to the plurality of locations from the detected X-rays; creating a plurality of sectional images by reconstructing the plurality of projected images; determining at least one of the plurality of sectional images having a preset feature in a region of interest; and inserting an indicator to indicate the feature to the at least one sectional image.

The X-ray imaging apparatus may further include a display, and the control method of the X-ray imaging apparatus may further include controlling the display to display the at least one sectional image to which the indicator is inserted.

The preset feature may include at least one type of a foreign body in a joint, a joint cartilage defect, bone fractures, a joint having maximum thickness, or a joint having minimum thickness.

The determining of the at least one sectional image may include performing image processing on the region of interest of each of the plurality of sectional images to determine whether there is the preset feature in each of the plurality of sectional images.

The performing of the image processing on the region of interest of each of the plurality of sectional images may include separating a joint image and a bone image included in the region of interest in each of the plurality of sectional images.

The performing of the image processing on the region of interest of each of the plurality of sectional images may include determining at least one of whether there is a foreign body in the joint, whether there is a joint cartilage defect, or whether there is bone fracture in each of the plurality of sectional images based on image processing on the joint image and the bone image in each of the plurality of sectional images.

The performing of the image processing on the region of interest of each of the plurality of sectional images may include measuring joint thickness in the region of interest in each of the plurality of sectional images based on the joint image and the bone image in each of the plurality of sectional images,

The inserting of the indicator to indicate the feature to the at least one sectional image may include determining at least one of a sectional image having maximum joint thickness or a sectional image having minimum joint thickness among the plurality of sectional images, and inserting an indicator to indicate a joint to the determined sectional image.

The determining of the at least one sectional image may include performing a neural network operation on each of the plurality of sectional images, and determining whether there is the preset feature in each of the plurality of sectional images based on information resulting from the neural network operation for the sectional image.

The X-ray imaging apparatus may further include an input device receiving an input of a user, and the control method of the X-ray imaging apparatus may further include controlling the display to display one of the plurality of sectional images, and receiving a choice of at least one region of interest including a portion of interest in the sectional image from the user through the input device.

The control method of the X-ray imaging apparatus may further include, when a preset input is received from the user through the input device, controlling the display to display the at least one of the plurality of sectional images to which the indicator is inserted.

The control method of the X-ray imaging apparatus may further include performing image processing on one of the plurality of sectional images to determine a joint included in the sectional image, and determining a region of interest to include the determined joint.

The control method of the X-ray imaging apparatus may, when there exist a plurality of previous sectional images of the subject including the region of interest and previously obtained, controlling the display to display at least one previous sectional image with an indicator inserted to the region of interest among the plurality of previous sectional images and at least one sectional image to which the indicator is inserted.

The control method of the X-ray imaging apparatus may further include determining whether there is a change in the feature by comparing the at least one sectional image with the at least one previous sectional image, and controlling the display to display information about the determination of whether there is a change in the feature.

### BRIEF DESCRIPTION OF THE DRAWINGS

The above and other aspects of the disclosure will become more apparent to those of ordinary skill in the art by describing in detail example embodiments thereof with reference to the accompanying drawings, in which:
FIG. 1 is a control block diagram of an X-ray imaging apparatus, according to an embodiment of the disclosure;
FIG. 2 is an external view illustrating a configuration of and X-ray imaging apparatus, according to an embodiment of the disclosure;
FIG. 3 is an external view illustrating an X-ray imaging apparatus controlling the position of an X-ray source to obtain projected images from a plurality of locations, according to an embodiment of the disclosure;
FIG. 4 shows a sectional image of a subject created by an X-ray imaging apparatus, according to an embodiment of the disclosure;
FIG. 5 shows an occasion when an X-ray imaging apparatus receives a region of interest from the user, according to an embodiment of the disclosure;
FIG. 6 shows an occasion when an X-ray imaging apparatus applies an image segmentation algorithm to a region of interest, according to an embodiment of the disclosure;
FIG. 7 shows an occasion when an X-ray imaging apparatus inserts an indicator to indicate a feature to a sectional image having the feature among a plurality of sectional images, according to an embodiment of the disclosure;
FIG. 8 shows an occasion when an X-ray imaging apparatus displays a sectional image with an indicator inserted thereto, according to an embodiment of the disclosure;
FIG. 9 shows an occasion when an X-ray imaging apparatus calculates a numerical value for each region of interest based on a state of a feature, according to an embodiment of the disclosure;
FIG. 10 shows an occasion when an X-ray imaging apparatus displays a calculation ground for a numerical value for a region of interest, according to an embodiment of the disclosure;
FIG. 11 shows preset calculation criteria, according to an embodiment of the disclosure;
FIG. 12 shows an occasion when an X-ray imaging apparatus displays a previous sectional image and a current sectional image for comparison, according to an embodiment of the disclosure; FIG. 13 shows an occasion when an X-ray imaging apparatus displays a previous sectional image and a current sectional image for comparison, according to an embodiment of the disclosure;
FIG. 14 is a flowchart of an instance of inserting an indicator to indicate a feature to a sectional image having the feature in a control method of an X-ray imaging apparatus, according to an embodiment of the disclosure;
FIG. 15 is a flowchart of an instance of displaying a numerical value calculated based on a state of a feature in a control method of an X-ray imaging apparatus, according to an embodiment of the disclosure; and
FIG. 16 is a flowchart of an instance of comparing a previously captured sectional image with a current sectional image in a control method of an X-ray imaging apparatus, according to an embodiment of the disclosure.

### DETAILED DESCRIPTION

Embodiments and features as described and illustrated in the disclosure are merely examples, and there may be various modifications replacing the embodiments and drawings at the time of filing this application.

It will be further understood that the term "connect" or its derivatives refer both to direct and indirect connection, and the indirect connection includes a connection over a wireless communication network.

The terminology used herein is for the purpose of describing particular embodiments only and is not intended to limit the disclosure. It is to be understood that the singular forms "a," "an," and "the" include plural references unless the context clearly dictates otherwise. It will be further understood that the terms "comprise" and/or "comprising," when used in this specification, specify the presence of stated features, integers, steps, operations, elements, and/or components, but do not preclude the presence or addition of one or more other features, integers, steps, operations, elements, components, and/or groups thereof.

The terms including ordinal numbers like "first" and "second" may be used to explain various components, but the components are not limited by the terms. The terms are only for the purpose of distinguishing a component from another. Thus, a first element, component, region, layer or chamber discussed below could be termed a second element, component, region, layer or section without departing from the teachings of the disclosure.

Furthermore, the terms, such as "∼ part", "∼ block", "∼ member", "∼ module", etc., may refer to a unit of handling at least one function or operation. For example, the terms may refer to at least one process handled by hardware such as field-programmable gate array (FPGA)/application specific integrated circuit (ASIC), etc., software stored in a memory, or at least one processor.

Reference numerals used for method steps are just used to identify the respective steps, but not to limit an order of the steps. Thus, unless the context clearly dictates otherwise, the written order may also be practiced otherwise.

Reference will now be made in detail to embodiments, which are illustrated in the accompanying drawings, wherein like reference numerals refer to the like elements throughout.

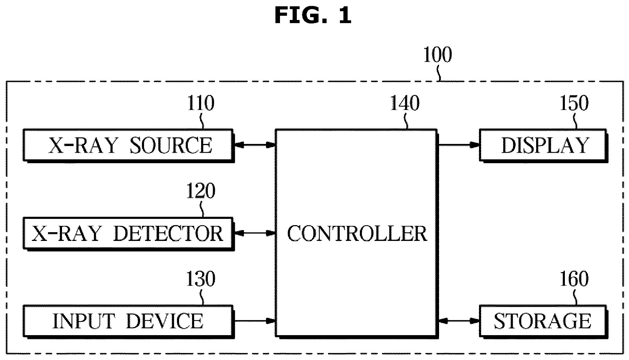

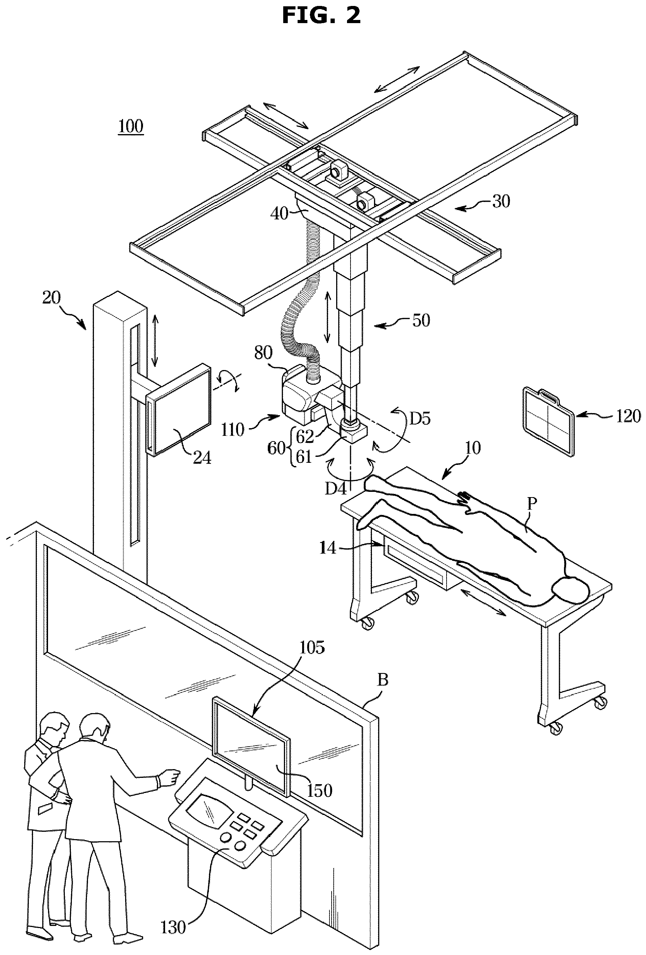

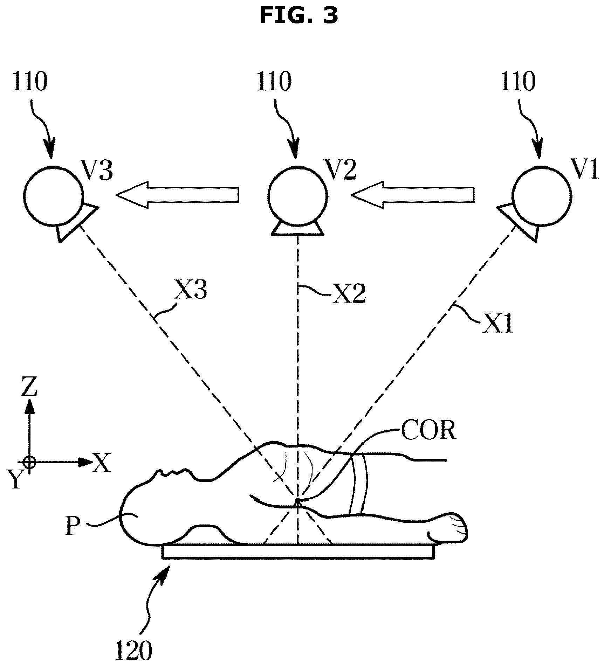

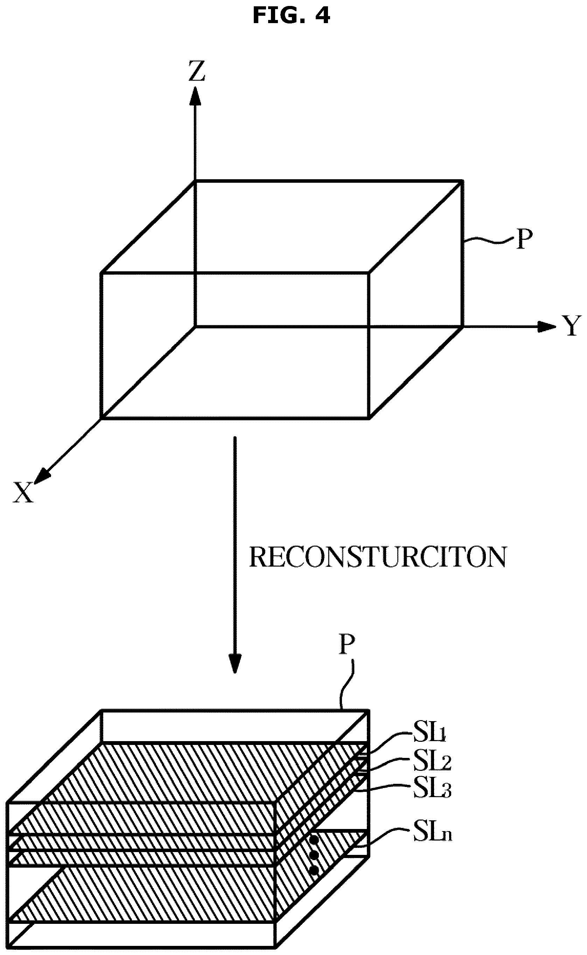

FIG. 1 is a control block diagram of an X-ray imaging apparatus, according to an embodiment of the disclosure, FIG. 2 is an external view illustrating a configuration of and X-ray imaging apparatus, according to an embodiment of the disclosure, FIG. 3 is an external view illustrating an X-ray imaging apparatus controlling the position of an X-ray source to obtain projected images from a plurality of locations, according to an embodiment of the disclosure, and FIG. 4 shows a sectional image of a subject created by an X-ray imaging apparatus, according to an embodiment of the disclosure.

Referring to FIGS. 1 and 2, an X-ray imaging apparatus 100 according to an embodiment of the disclosure may include an X-ray source 110 for irradiating X-rays to a subject from a plurality of locations, an X-ray detector 120 for detecting X-rays that has been irradiated from the plurality of locations and passed through the subject, an input device 130 for receiving an input from the user, a controller 140 for obtaining a plurality of projected images corresponding to the plurality of locations from the detected X-rays, creating a plurality of sectional images by reconstructing the plurality of projected images, determining whether there is a feature in a region of interest in each of the plurality of sectional images, which represents a disease, and inserting an indicator to indicate the determined feature to the sectional image or determining a numerical value that scores a disease based on a state of the determined feature, a display 150 for displaying a sectional image to which an indicator to indicate a feature is inserted or to which a numerical value for a region of interest is inserted, and a storage 160 for storing various information required to control the X-ray imaging apparatus 100.

A guide rail 30 may be installed on the ceiling of an examination room where an X-ray imaging apparatus 100 is placed, and an X-ray source 110 linked to a moving carriage 40 that moves along the guide rail 30 may be moved to a position corresponding to a subject P.

The moving carriage 40 and the X-ray source 110 may be linked through a foldable post frame 50 to adjust the altitude of the X-ray source 110.

A rotary joint 60 is arranged between the X-ray source 110 and the post frame 50. The rotary joint 60 may include a first rotary joint 61 coupled to the post frame 50 and a second rotary joint 62 coupled to the X-ray source 110.

The first rotary joint 61 may be rotated in a fourth direction D4 and the second rotary joint 62 may be rotated in a fifth direction D5. By rotating the second rotary joint 62 in the fifth direction D5, a tilt angle or a rotation angle of the X-ray source 110 may be adjusted.

The X-ray source 110 may be moved automatically or manually. In the former case, the X-ray imaging apparatus 100 may further include a driver, such as a motor to provide power to move the X-ray source 110.

An X-ray detector 120 may be implemented as a fixed type of X-ray detector fixed on a stand 20 or a table 10, or may detachably equipped in an install part 14, 24. Alternatively, the X-ray detector 200 may be implemented as a portable X-ray detector available at any place. The portable X-ray detector may be implemented in a wired type or a wireless type depending on a data transmission method and a power supplying method.

In the embodiment of the disclosure, a mode in which X-raying is performed with the X-ray detector 120 installed in an install part 14 of the imaging table 10 is called a table mode; a mode in which X-raying is performed with the X-ray detector 120 installed in an install part 24 of the imaging stand 20 is called a stand mode; a mode in which X-raying is performed with the X-ray detector 120 not installed in the install part 14, 24 but located behind an imaging portion of the subject is called a portable mode.

The X-ray detector 120 mounted on the install part 14, 24 may be moved automatically or manually. In the former case, the X-ray imaging apparatus 100 may further include a driver, such as a motor to provide power to move the install part 14, 24.

The X-ray detector 120 may or may not be included as an element of the X-ray imaging apparatus 100. In the latter case, the X-ray detector 120 may be registered in the X-ray imaging apparatus 100 by the user. Furthermore, in both cases, X-ray data obtained by the X-ray detector 120 detecting X-rays may be sent to the workstation 105.

A sub user interface 80 may be arranged on one side of the X-ray source 110 to provide information for the user and receive a command from the user, and may perform a part or all of the functions performed by the input 130 and the display 150 of the workstation 180.

In an embodiment, the X-ray imaging apparatus 100 may be implemented as tomosynthesis equipment, which takes images of the subject from different angles, different views, or different locations while moving the X-ray source 110, thereby obtaining sectional images (tomographic images) or three-dimensional (3D) data of the subject.

Referring to FIG. 3, when a portion to be scanned (hereinafter, referred to as a scanning portion) of a subject is located on top of the X-ray detector 120, the X-ray source 110 may rotate around the scanning portion and irradiate X-rays X1, X2, and X3 thereto from a plurality of views V1, V2, and V3, thereby obtaining a plurality of projection images.

In this regard, the rotation of the X-ray source 110 may be performed by linear motion along the guide rail 30 and by changing tilt angles or rotation angles using the rotary joint 62.

The center of rotation (COR) of the X-ray source 110 may be set to be the center of the scanning portion. Accordingly, the COR on the z-axis varies by thickness of the scanning portion. The x-, y-, and z-axes are relative to each other, and in this embodiment, a plane parallel to the plane of the X-ray detector 120 corresponds to the xy-plane and the z-axis corresponds to the incidence direction of an X-ray or the thickness direction of a subject P.

In this case, the COR may be different depending on a scanning portion of the subject P because of a difference in thickness between types of the scanning portions (e.g., a hand, a foot, an abdominal region, etc.) of the subject.

Even for the same scanning portion, the COR of the X-ray source 110 may differ by imaging protocol. For example, even in the case of scanning the same abdominal region, the thickness of the subject with respect to the incidence direction of the X-ray may differ from the anterior posterior (AP) protocol according to which the subject is scanned from the front to the lateral protocol according to which the subject is scanned from the side, so the COR of the X-ray source 110 may differ as well.

As such, the X-ray source 110 may irradiate X-rays from a plurality of locations. The X-ray detector 120 may then detect X-rays that has been irradiated from the plurality of locations, i.e., from different angles, and has passed a subject, and the controller 140 may obtain a plurality of projected images corresponding to the plurality of locations. Although FIG. 3 shows X-ray irradiation from three locations, the location of the X-ray source 10 is not limited thereto and there is no limitation on the number of the locations.

Each of the plurality of projected images may correspond to projected data from a corresponding location.

For this, the X-ray source 110 may be equipped with an X-ray tube for generating an X-ray and a collimator for adjusting an irradiation area of the X-ray generated by the X-ray tube. Accordingly, the X-ray source 110 may also be called a tube head unit (THU).

To obtain the projected image, the X-ray detector 120 may detect an X-ray that has been irradiated from a plurality of locations and has passed through the subject P.

For this, the X-ray detector 120 may include a light receiving element for detecting an X-ray and converting the X-ray into an electric signal, and a readout circuit for reading out the electric signal. The light receiving element may be formed with a single crystal semiconductor material, such as Ge, CdTe, CdZnTe or GaAs. The readout circuit may be formed of a two dimensional (2D) pixel array including a plurality of pixels, each of which may be coupled to the light receiving element. This structure is, however, an example of the X-ray detector 120 without being limited thereto.

Furthermore, the X-ray detector 120 may send an electric signal output from each pixel to the controller 140, which may in turn create a projected image based on the electric signal. As described above, the X-ray source 110 may irradiate X-rays to the subject P from the plurality of locations. Accordingly, the controller 140 may create a projected image corresponding to each location of the X-ray source 110.

A workstation 105 may be provided in the space separated by a blackout curtain B from the space where the X-ray source 110 is placed. The workstation 105 may be equipped with an input 130 for receiving commands from the user and a display 150 for displaying information.

The input device 130 may receive an input from the user.

Specifically, the input device 130 may receive an input to set up a region of interest from the user. The region of interest may correspond to an area in which to determine whether there is a feature, i.e., a disease. For example, the region of interest may correspond to an area including a particular joint.

In this case, the controller 140 may set up an area input from the user through the input device 130 as the region of interest, and there may be one or more regions of interest set up according to the input. How to receive the input for establishing the region of interest will be described later in detail.

The input device 130 may also receive a choice of the established region of interest. Specifically, the input device 130 may receive a choice of a particular region of interest from among the established regions of interest.

In this case, according to an embodiment of the disclosure, the X-ray imaging apparatus 100 may display a sectional image with an indicator to indicate the feature in the region of interest inserted thereto among the plurality of sectional images through the display 150.

Furthermore, in an embodiment of the disclosure, the X-ray imaging apparatus 100 may display the sectional image having the feature in the chosen region of interest among the plurality of sectional images and at least one of a numerical value calculated based on a state of the feature and the calculation ground on the display 150.

How to receive the choice of the particular region of interest from among the established regions of interest will be described later in detail.

For this, the input device 130 may be arranged in the main body of the X-ray imaging apparatus 100 and implemented with mechanical buttons, knobs, a touch pad, a touch screen, a stick-type manipulation device, a trackball, or the like. The input device 130 provided as a touch pad or touch screen may be arranged on the display 150.

Furthermore, the input device 130 may be provided as a separate input device, such as a key board, mouse, or the like, which is wiredly or wirelessly connected to the X-ray imaging apparatus 100 or to a workstation wiredly or wirelessly coupled with the X-ray imaging apparatus 100.

In an embodiment, the controller 140 may obtain a plurality of projected images corresponding to a plurality of locations from the detected X-ray, as shown in FIG. 4, and create a plurality of sectional images SL₁, SL₂, SL₃, ..., SLₙ by reconstructing the plurality of projected images.

Specifically, the X-ray source 110 obtains projected images corresponding to projected data of the subject P from different viewpoints by rotating around the subject P at regular angles, and the controller 140 may create the sectional images SL₁, SL₂, SL₃, ..., SLₙ of the subject P by reconstructing the projected images, as shown in FIG. 4.

The sectional images SL₁, SL₂, SL₃, ..., SLₙ may correspond to tomosynthesis images created according to the tomosynthesis method, and slices in medical imaging areas.

For a method of creating a sectional image by reconstructing a projected image, there may be iterative reconstruction, matrix inversion, back-projection, Fourier transform, filtered back-projection, etc.

The iterative reconstruction is a method of continuously correcting the projected image until data approximately equal to an original structure of the subject P is obtained, the back-projection is a method of restoring the projected images obtained from a plurality of viewpoints on a single screen, and the Fourier transform is a method of transforming the projected image from the spatial area to the frequency area. The filtered back-projection is a method of back-projecting a projected image after performing filtering process to cancel a blur formed around a center portion of the projected image.

However, the method of creating a sectional image by reconstructing a projected image is not limited to the above example, and any method known to the public may be applied without limitation.

For example, when the X-ray source 110 scans the subject P while rotating around a rotational axis, the Y-axis, the controller 140 may create n (n is an integer equal to or greater than 2) sectional images SL1, SL2, SL3,..., SLₙ parallel to the XY-plane along the Z-axis by reconstructing the projected image obtained by the X-ray detector 120, as shown in FIG. 4.

In this case, the controller 140 may allocate an identification number for each of the plurality of sectional images to distinguish one from another.

In an embodiment of the disclosure, the controller 140 may determine whether there is a feature representing a disease in a region of interest in each of the plurality of sectional images, and insert an indicator to indicate the feature to the sectional image or determine a numerical value that scores the disease based on a state of the feature.

As the plurality of sectional images are created by changing the depth in the direction of thickness of the subject P, the feature may be detected in a particular sectional image but not detected in some other sectional image(s). That is, as the plurality of sectional images are captured from different depths in the direction of thickness, each of them may or may not have a feature and if there is a feature, the feature type may be different.

The controller 140 may determine at least one sectional image having a preset feature in the region of interest among the plurality of sectional images.

In other words, the controller 140 may determine whether there is the preset feature in the region of interest for each of the obtained plurality of sectional images.

The region of interest may be set up based on the user's input through the input device 130, as described above.

Alternatively, the region of interest may be set up by the controller 140 without the user's input. Specifically, the controller 140 may classify a particular portion (e.g., joints) that is likely to have a disease based on an image process, e.g., edge detection, morphological operation, or the like performed on the obtained sectional image, and establish an area including the classified particular portion as the region of interest. The particular portion may be preset in a design stage or set by the user through the input device 130.

The preset feature may include at least one type of a foreign body in a joint, a joint cartilage defect, bone fractures, a joint having maximum thickness, or a joint having minimum thickness. For example, the feature may correspond to a disease such as a foreign body, a defect, fracture, or the like, or correspond to properties of joint thickness.

The joint having the maximum thickness may correspond to a joint having the widest thickness among joint thicknesses measured in the respective regions of interest of the plurality of sectional images. The joint having the minimum thickness may correspond to a joint having the narrowest thickness among joint thicknesses measured in the respective regions of interest of the plurality of sectional images.

The thickness of the joint may correspond to thickness of a joint that exists in the region of interest. Specifically, the thickness of the joint may correspond to thickness of a joint between bones or a gap between the bones in the region of interest.

The controller 140 may perform image processing on the region of interest of each of the plurality of sectional images to determine whether there is the preset feature in each of the plurality of sectional images.

Furthermore, the controller 140 may perform a neural network operation on each of the plurality of sectional images and determine whether there is the preset feature in each of the plurality of sectional images based on information resulting from the neural network operation for the sectional image.

How to determine whether there is a feature in each of the plurality of sectional images using image processing or the neural network operation will be described later in detail.

In an embodiment of the disclosure, the controller 140 may insert an indicator to indicate the detected feature to each of the at least one sectional image determined to have the preset feature.

Specifically, the controller 140 may insert the indicator to the sectional image to make the sectional image stand out among the plurality of sectional images, and the indicator may be inserted to a point corresponding to the feature.

Furthermore, the controller 140 may control the display 150 to display the at least one sectional image with the indicator inserted thereto. In this case, the controller 140 may control the display 150 to display both sectional images with or without the indicator inserted thereto, or control the display 150 to display only the sectional image with the indicator inserted thereto among the plurality of sectional images.

In the latter case, the controller 140 may control the display 150 to display only the sectional image with the indicator inserted thereto when receiving a preset input through the input device 130 in some embodiments of the disclosure.

For example, the controller 140 may control the display 150 to display at least one of the plurality of sectional images, and control the display 150 to display a sectional image in which the feature is found in a particular region of interest when receiving a choice of the particular region of interest from the user. In this case, the sectional image displayed on the display 150 may include the indicator to indicate the feature.

This may enable the user to receive a particular sectional image from which the user is able to tell the feature without need to check every one of the plurality of sectional images, and to determine more easily whether there is the feature through the indicator to indicate the feature.

Furthermore, the controller 140 may control the display 150 to match and display each type of preset feature and an identification number of the sectional image corresponding to the type.

For example, the controller 140 may control the display 150 to display matching of an identification number of a sectional image determined to have a particular type of preset feature with information about the particular type.

In another embodiment of the disclosure, the controller 140 may determine a numerical value that scores a disease based on a state of the determined feature.

Specifically, the controller 140 may determine a numerical value for the region of interest based on a state of a feature in at least one sectional image determined to have the feature. The region of interest may be set up in the singular or plural according to preferences.

In this regard, the controller 140 may determine the numerical value by using information about correlations between features and numerical values or based on information resulting from a neural network operation.

The controller 140 may calculate a numerical value for the region of interest in each of the at least one sectional image based on information about correlations between features and numerical values and respective feature in the at least one sectional image, and determine a numerical value corresponding to the region of interest by summing the calculated numerical values.

Alternatively, the controller 140 may perform a neural network operation on each of at least one sectional image determined to have a feature, calculate a numerical value for the region of interest of each of the at least one sectional image based on information resulting from the neural network operation for the sectional image, and determine a numerical value for the region of interest by summing the calculated numerical values.

Furthermore, the controller 140 may control the display 150 to display at least one of numerical values for the respective regions of interest and a sum of the numerical values on one of the plurality of sectional images.

This may enable the user to determine whether the subject P has a disease and the extent of the disease for each region of interest by checking the numerical value that scores the disease of the subject P.

Furthermore, when receiving a choice of one of regions of interest from the user through the input device 1subject P, the controller 140 may control the display 150 to display at least one of a sectional image having the feature in the chosen region of interest and a calculation ground for the numerical value for the chosen region of interest.

This may enable the user to receive a particular sectional image from which the user is able to tell the feature without need to check every one of the plurality of sectional images, and to determine a disease of the subject P more easily based on the calculation ground for the numerical value.

In an embodiment of the disclosure, the controller 140 may determine whether there is a previous sectional image previously obtained for the subject P for which the plurality of sectional images are obtained.

Specifically, based on the patient information of the subject P, the controller 140 may determine whether there is a sectional image that corresponds to the patient information. In other words, on an occasion when an examination is currently performed on the subject P to obtain a plurality of sectional images, the controller 140 may determine whether there was a previous examination made on the subject P that obtained a sectional image.

In this case, the controller 140 may determine whether there is a previous sectional image of the subject P by comparing patient information assigned to a sectional image stored in the storage 160 with patient information for the subject P.

Alternatively, the controller 140 may determine whether there is a sectional image that corresponds to the patient information for the subject P among the sectional images stored, by communication with a PACS server that receives and stores sectional images obtained from the X-ray imaging apparatus 100 through a communicator (not shown).

In this regard, when there exist a plurality of previous sectional images that include a region of interest corresponding to the current examination on the subject P and that were previously obtained, the controller 140 may control the display 150 to display at least one previous sectional image with the indicator inserted to the region of interest among the plurality of previous sectional images and at least one sectional image with the indicator inserted thereto in the current examination.

This may enable the user to receive a sectional image from the previous examination on the subject P as well as a sectional image from the current examination without need to search the sectional image from the previous examination, and to determine progress of the disease of the subject P more easily by comparing the sectional image from the current examination with the sectional image from the previous examination.

Furthermore, the controller 140 may determine whether there is a change in the feature by comparing at least one sectional image from the current examination with at least one sectional image from the previous examination.

Specifically, the controller 140 may determine at least one of whether there is a change in presence or absence of the feature, whether there is a change in thickness of a joint among the features, or whether there is a change in numerical value based on a state of the feature.

More specifically, the controller 140 may extract a feature from a previous sectional image based on an image process or neural network operation on the previous sectional image, and compare it with the feature in the sectional image from the current examination to determine at least one of whether there is a change in presence or absence of the feature, whether there is a change in thickness of a joint among the features, or whether there is a change in numerical value based on a state of the feature.

Subsequently, the controller 140 may control the display 150 to display information about the change determined in the feature. This may enable the user to determine progress of the disease of the subject P more easily.

The controller 140 may include at least one memory for storing a program for carrying out the aforementioned and following operations, and at least one processor for executing the program.

In an embodiment, the display 150 may display a sectional image obtained, a sectional image with the indicator inserted thereto, or a sectional image with the numerical value for the region of interest inserted thereto.

Specifically, the display 150 may display one of the plurality of sectional images obtained, under the control of the controller 140.

Accordingly, the user may set up a region of interest in a sectional image displayed on the display 150 through the input device 130.When there are a plurality of previous sectional images previously obtained, a sectional image displayed may correspond to a previous sectional image with an indicator to indicate the feature inserted thereto.

Otherwise, when no sectional image has been previously obtained, a sectional image displayed may correspond to a sectional image corresponding to a preset location. For example, the preset location may correspond to a location where a joint that is more likely to have a disease is captured more clearly than other locations, and may be set in a design stage of the X-ray imaging apparatus 100 or set by the user though the input device 130. The sectional image displayed is not, however, limited thereto, and may correspond to any of the plurality of sectional images in some embodiments of the disclosure.

Furthermore, the display 150 may display a sectional image with the indicator inserted thereto, or a sectional image with the numerical value for the region of interest inserted thereto, as described above.

Moreover, the display 150 may display both a sectional image from the previous examination and a sectional image from the current examination, as described above, and in some embodiments, may display information about whether there is a change in the feature.

For this, the display 150 may be provided in the main body of the X-ray imaging apparatus 100, provided as a separate display wiredly or wirelessly connected to the X-ray imaging apparatus 100, or provided in a workstation wiredly or wireless coupled with the X-ray imaging apparatus 100.

The display 150 may include e.g., a Liquid Crystal Display (LCD), a Light Emitting Diode (LED) display, an organic LED (OLED) display, Micro-Electromechanical System (MEMS) display, or an electronic paper display. Types of the display 150 are not, however, limited thereto, and may include any type of display capable of displaying a sectional image for the user.

In an embodiment of the disclosure, the storage 160 may store various information required to control the X-ray imaging apparatus 100. For example, the storage 160 may store a previous sectional image of the subject P, information about an image processing algorithm to determine a feature, information about correlations between features and numerical values, etc.

The storage 160 may be implemented with at least one of a non-volatile memory device, such as cache, read only memory (ROM), programmable ROM (PROM), erasable programmable ROM (EPROM), electrically erasable programmable ROM (EEPROM), and a flash memory, a volatile memory device, such as random access memory (RAM), or a storage medium, such as hard disk drive (HDD) or compact disk ROM (CD-ROM), without being limited thereto. It is not, however, limited thereto, and may have any type of storage that is capable of storing various information.

The storage 160 may be a memory implemented with a chip separate from the aforementioned processor in relation to the controller 140, or may be implemented with the processor in a single chip.

How to set up a region of interest for a sectional image when the X-ray imaging apparatus 100 obtains a plurality of sectional images according to an embodiment of the disclosure will now be described in detail.

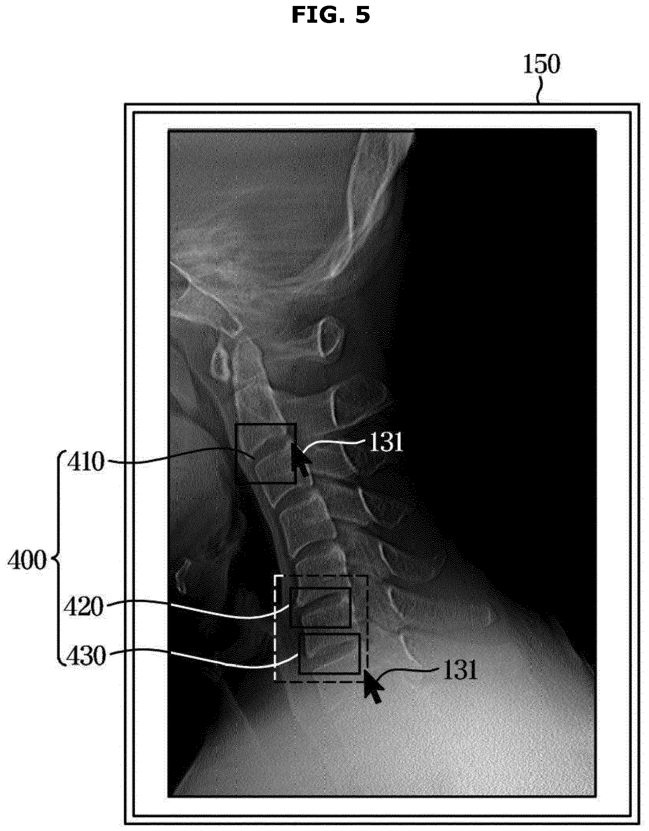

FIG. 5 shows an occasion when the X-ray imaging apparatus 100 receives a region of interest from the user, according to an embodiment of the disclosure.

Referring to FIG. 5, the controller 140 may control the display 150 to display one of a plurality of sectional images.

Accordingly, the user may set up a region of interest 400 in a sectional image displayed on the display 150 through the input device 130.When there are a plurality of previous sectional images previously obtained, a sectional image displayed may correspond to a previous sectional image with an indicator to indicate the feature inserted thereto.

Otherwise, when no sectional image has been previously obtained, the sectional image displayed may correspond to a sectional image from a preset location. For example, the preset location may correspond to a location where a joint that is more likely to have a disease is captured more clearly than other locations, and may be set in a design stage of the X-ray imaging apparatus 100 or set by the user though the input device 130. The sectional image displayed is not, however, limited thereto, and may correspond to any of the plurality of sectional images in some embodiments of the disclosure.

Specifically, the input device 130 may receive an input to set up a region of interest from the user. The region of interest may correspond to an area in which to determine whether there is a feature, i.e., a disease. For example, the region of interest may correspond to an area including a particular joint.

In this case, the controller 140 may set up an area input from the user through the input device 130 as the region of interest 400, and there may be one or more regions of interest 400 set up according to the input.

For example, when receiving a choice of the particular portion, e.g., a joint, through the input device 130 (e.g., by clicking on a point 131), the controller 140 may set up a certain area including the portion as a region of interest 410. Furthermore, the controller 140 may receive a choice of each of a plurality of portions through the input device 130 and set up a certain area including each of the plurality of portions as a region of interest 400.

For example, when receiving a choice of an area including a plurality of portions through the input device 130 (e.g., by dragging from the point 131), the controller 140 may set up certain areas including the plurality of portions as regions of interest 420 and 430. Although FIG. 5 shows two portions as the plurality of portions, it is merely an example and there is no limitation on the number of inputs for the region of interest 400 through the input device 130.

Alternatively, unlike what is shown in FIG. 5, the region of interest may be set up by the controller 140 without intervention of the user. Specifically, the controller 140 may classify a particular portion (e.g., a joint) that is likely to have a disease based on an image process, e.g., edge detection, morphological operation, or the like performed on the obtained sectional image, and establish an area including the classified particular portion as the region of interest 400. The particular portion may be preset in a design stage or set by the user through the input device 130.

How the X-ray imaging apparatus 100 determines whether there is a feature in the region of interest in each of the plurality of sectional images and inserts an indicator to indicate the feature to the sectional image determined to have the feature will now be described in detail.

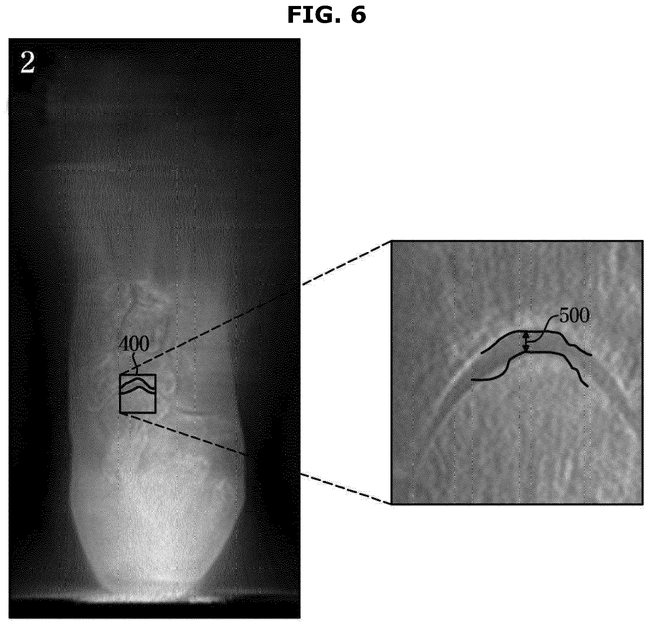

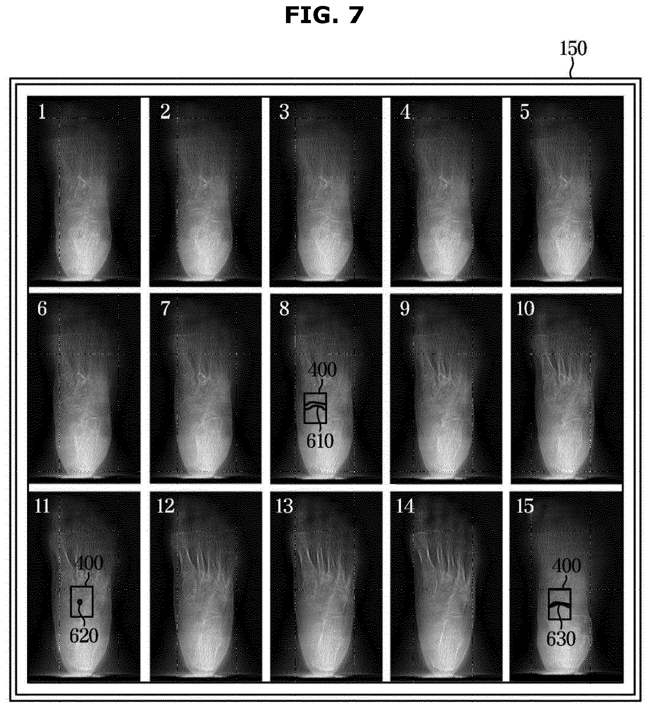

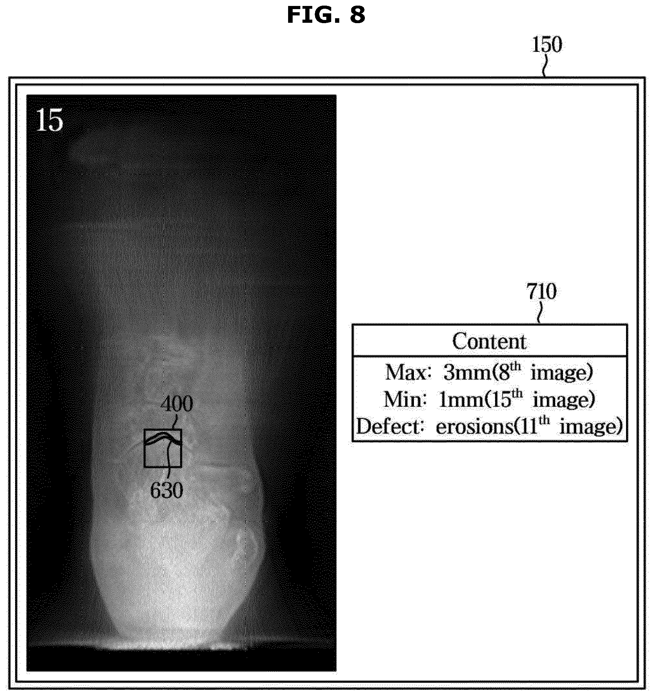

FIG. 6 shows an occasion when the X-ray imaging apparatus 100 applies an image segmentation algorithm to the region of interest, according to an embodiment of the disclosure, FIG. 7 shows an occasion when an X-ray imaging apparatus 100 inserts an indicator to indicate a feature to a sectional image having the feature among a plurality of sectional images, according to an embodiment of the disclosure, and FIG. 8 shows an occasion when the X-ray imaging apparatus 100 displays a sectional image with an indicator inserted thereto, according to an embodiment of the disclosure.

Referring to FIG. 6, the controller 140 may determine whether there is a feature for each of a plurality of sectional images corresponding to a plurality of locations, according to an embodiment of the disclosure.

The controller 140 may perform image processing on the region of interest 400 of each of the plurality of sectional images to determine whether there is a preset feature in each of the plurality of sectional images.

The preset feature may include at least one type of a foreign body in a joint, a joint cartilage defect, bone fractures, a joint having maximum thickness, or a joint having minimum thickness. For example, the feature may correspond to a disease such as a foreign body, a defect, fracture, or the like, or correspond to properties of joint thickness.

The joint having the maximum thickness may correspond to a joint having the widest thickness among joint thicknesses measured in the respective regions of interest of the plurality of sectional images obtained corresponding to the plurality of locations. The joint having the minimum thickness may correspond to a joint having the narrowest thickness among joint thicknesses measured in the respective regions of interest of the plurality of sectional images obtained corresponding to the plurality of locations.

The thickness of the joint may correspond to thickness of a joint that exists in the region of interest. Specifically, the thickness of the joint may correspond to thickness of a joint between bones or a gap between the bones in the region of interest.

Specifically, the controller 10 may separate a joint image and a bone image included in the region of interest 400 of each of the plurality of sectional images. For example, referring to FIG. 6, the controller 140 may divide the region of interest 400 in a sectional image, e.g., a sectional image with identification number 2, into the joint image and the bone image based on an image segmentation algorithm. In light of the body structure, a joint is typically formed between bones, so the joint image in the region of interest 400 may be arranged between two or more bone images.

The controller 140 may determine at least one of whether there is a foreign body in the joint, whether there is a joint cartilage defect, or whether there is bone fracture in each of the plurality of sectional images based on image processing on the joint image and bone image in each of the plurality of sectional images. In other words, the controller 140 may determine whether there is a feature based on the joint image and the bone image separated from the region of interest 400.

In this regard, the controller 140 may extract a feature in the sectional image by applying an image processing algorithm such as an object recognition algorithm. For example, the controller 140 may extract the feature, e.g., a foreign body in the joint, a joint cartilage defect, bone fractures, included in the sectional image by applying edge detection. Alternatively, the controller 140 may extract the feature included in the sectional image by detecting the shape through morphological operation.

The image processing algorithm to extract a feature is not, however, limited thereto, and any image processing algorithm may be used without limitation as long as it may extract a feature included in a sectional image.

The controller 140 may determine a type of the feature extracted based on information about types corresponding to the feature extracted based on the image processing. The information about types corresponding to the feature may include information about a disease that may appear according to image characteristics.

In this way, the controller 140 may determine a sectional image having the feature in the region of interest among the plurality of sectional images based on the image processing.

Furthermore, the controller 140 may measure joint thickness 500 in the region of interest in each of the plurality of sectional images based on the joint image and the bone image in each of the plurality of sectional images.

Specifically, the controller 140 may measure thickness of a joint in the region of interest 400 in each of the plurality of sectional images based on the joint image and the bone image separated from the region of interest in each of the plurality of sectional images.

Specifically, the joint thickness 500 may correspond to thickness of a joint or a gap between bones in the region of interest.

Subsequently, the controller 140 may determine a joint having maximum thickness as a feature and a joint having minimum thickness as a feature based on thickness of the joint in the region of interest 400 in each of the plurality of sectional images.

The controller 140 may then determine the sectional images having the maximum joint thickness and having the minimum joint thickness among the plurality of sectional images as sectional images having features.

Furthermore, the controller 140 may perform a neural network operation on each of the plurality of sectional images and determine whether there is the preset feature in each of the plurality of sectional images based on information resulting from the neural network operation for the sectional image.

The information resulting from the neural network operation may include information about whether there is the feature in the region of interest in each of the plurality of sectional images.

The neural network refers to machine learning that embodies a neural structure capable of performing deep learning, and as weight and bias corresponding to elements of the neural network keep changing, learning confidence is improved.

Specifically, the X-ray imaging apparatus 100 may continue to receive sectional images and corresponding types of feature from the outside, and the controller 140 may keep on updating the weight, bias, and activation function included in the neural network based on the sectional image and the corresponding type of feature received from the outside, thereby improving the inference of the neural network. The X-ray imaging apparatus 100 may update the weight, bias, and activation function included in the neural network based on a sectional image and a type of feature corresponding to the sectional image determined by image processing.

The neural network may be stored in the storage 160 in the form of a computer program. In the following description, an operation processed by the neural network in a coded format of the computer program will be assumed, but the neural network is not limited to the stored computer program.

The neural network may include a convolution neural network (CNN) that generates a features map output by convolution of sectional images and enters the features map to the neural network, but it is not limited thereto and may be implemented with another deep learning algorithm including a recurrent neural network (RNN).

As such, the controller 140 may determine a sectional image having a feature in the region of interest 400 among the plurality of sectional images based on information resulting from image processing or neural network operation.

In an embodiment of the disclosure, the controller 140 may insert an indicator to indicate the detected feature to each of the at least one sectional image determined to have the preset feature.

Specifically, the controller 140 may insert the indicator to the sectional image to make the sectional image stand out among the plurality of sectional images, and the indicator may be inserted to a point corresponding to the feature.

For example, as shown in FIG. 7, the controller 140 may insert an indicator 610 to indicate a joint of maximum thickness to the sectional image (in this example, with identification number 8) having the joint of the maximum thickness in the region of interest 400 among the plurality of sectional images, insert an indicator 620 to indicate a joint cartilage defect to the sectional image (in this example, with identification number 11) having the joint cartilage defect in the region of interest 400 among the plurality of sectional images, and insert an indicator 630 to indicate a joint of minimum thickness to the sectional image (in this example, with identification number 15) having the joint of the minimum thickness in the region of interest 400 among the plurality of sectional images.

Furthermore, the controller 140 may control the display 150 to display the at least one sectional image with the indicator inserted thereto. In this case, the controller 140 may control the display 150 to display both sectional images with or without the indicator inserted thereto, or control the display 150 to display only the sectional image with the indicator inserted thereto among the plurality of sectional images.

For example, as shown in FIG. 7, the controller 140 may control the display 150 to display a plurality of sectional images and control the display 150 to display the sectional images with the indicators 610, 620, and 630 inserted thereto. This may enable the user of the X-ray imaging apparatus 100 to distinguish sectional images with features in the region of interest 400 among the plurality of sectional images. In another embodiment of the disclosure, as described above, unlike what is shown in FIG. 7, the controller 140 may control the display 150 to display only the sectional images with the indicator(s) inserted thereto among the sectional images.

In the latter case, the controller 140 may control the display 150 to display only the sectional image with the indicator inserted thereto when receiving a preset input through the input device 130 in some embodiments of the disclosure.

For example, the controller 140 may control the display 150 to display at least one of the plurality of sectional images, and control the display 150 to display a sectional image in which the feature is found in the particular region of interest 400, e.g., a sectional image with identification number 15 having a joint of minimum thickness, when receiving a choice of the particular region of interest 400 from the user, as shown in FIG. 8. In this case, the sectional image displayed on the display 150 may include the indicator 630 to indicate the feature.

This may enable the user to receive a particular sectional image from which the user is able to tell the feature without need to check every one of the plurality of sectional images, and to determine more easily whether there is the feature through the indicator to indicate the feature.

Furthermore, the controller 140 may control the display 150 to match and display each type of preset feature and an identification number of the sectional image corresponding to the type.

For example, the controller 140 may control the display 150 to display matching of an identification number of a sectional image determined to have a particular type of preset feature with information about the particular type. For example, when joint thickness is one of the types of feature, the controller 140 may control the display 150 to display information about the thickness of the joint as well.

Specifically, the display 150 may display a sectional image having a feature in the region of interest 400 as well as information 710 about the type of the feature in the sectional image.

For example, as shown in FIG. 8, the display 150 may display information indicating that a sectional image having a joint of maximum thickness in the region of interest 400 corresponds to the sectional image with the identification number 8, information indicating that a sectional image having a joint of minimum thickness in the region of interest 400 corresponds to the sectional image with the identification number 15, information indicating that a sectional image having a joint cartilage defect in the region of interest 400 corresponds to the sectional image with the identification number 11. Furthermore, the display 150 may also display information about thickness of the joint, e.g., a maximum thickness: 3mm, a minimum thickness: 1mm.

This may enable the user to identify a sectional image having a feature among the plurality of sectional images and determine a disease of the subject P more quickly and correctly.

In the following description, how the X-ray imaging apparatus 100 determines a numerical value for the region of interest 400 based on a state of a feature in the at least one sectional image determined to have the feature will be described in detail.

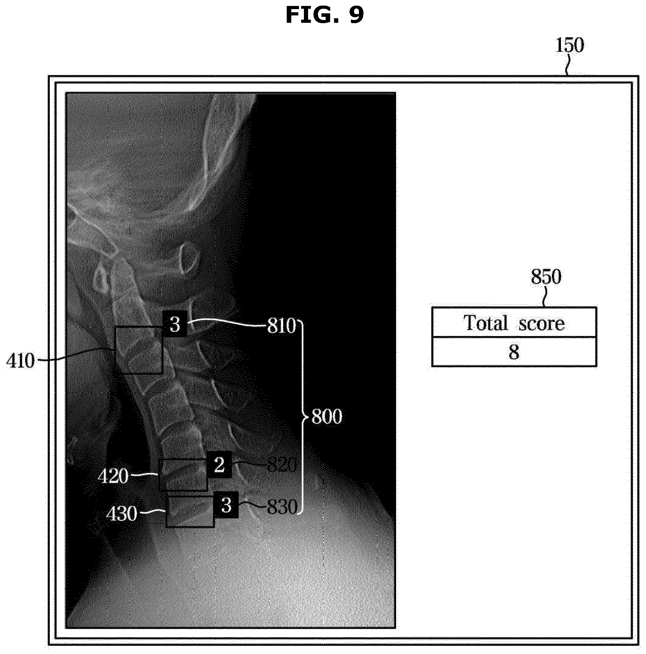

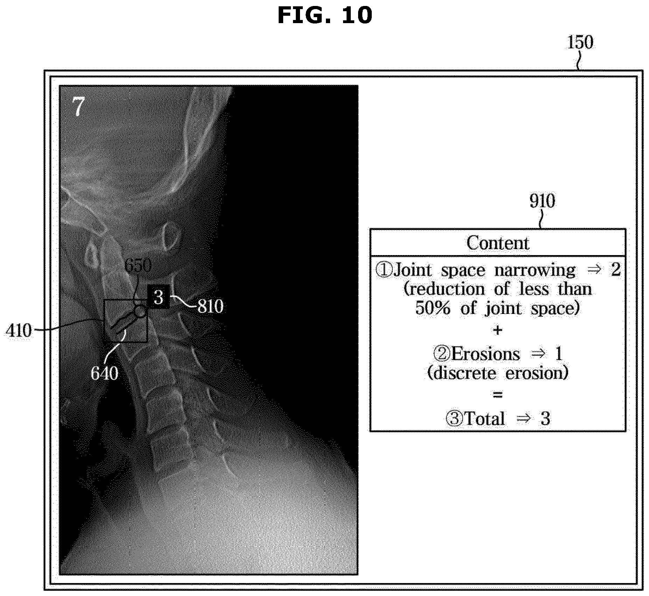

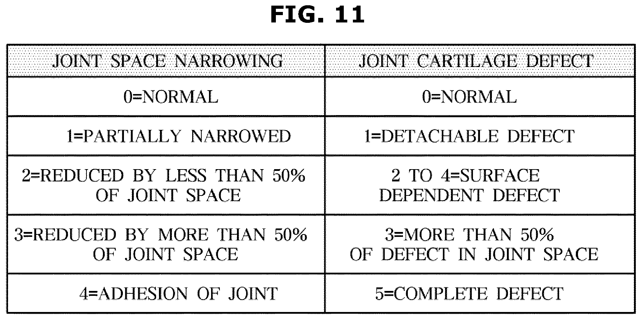

FIG. 9 shows an occasion when the X-ray imaging apparatus 100 calculates a numerical value for each region of interest 400 based on a state of a feature, according to an embodiment of the disclosure, FIG. 10 shows an occasion when the X-ray imaging apparatus 100 displays a calculation ground for a numerical value for the region of interest, according to an embodiment of the disclosure, and FIG. 11 shows preset calculation criteria, according to an embodiment of the disclosure.

Referring to FIG. 9, the controller 140 may determine a numerical value 800 for the region of interest 400 based on a state of a feature in at least one sectional image determined to have the feature, and control the display 150 to display the numerical value 800.

Specifically, the controller 140 may determine the numerical value 800 for the region of interest 400 based on a state of a feature in at least one sectional image determined to have the feature. The region of interest 400 may be set up in the singular or plural based on preferences.

In this regard, the controller 140 may determine the numerical value 800 by using information about correlations between features and numerical values or based on information resulting from a neural network operation. The information about correlations between features and numerical values may include information about types of features and numerical values based on the extent of the types of features.

For example, the controller 140 may calculate numerical values for the region of interest 400 of each of at least one sectional image based on information about correlations between features and numerical values and the respective feature in the at least one sectional image, and determine the numerical value 800 corresponding to the region of interest 400 by summing the calculated numerical values.

Specifically, the controller 140 may calculate numerical values for the region of interest of each of the at least one sectional image having the feature, and determine the numerical value 800 for the region of interest 400 by summing the calculated numerical values. For example, the controller 140 may calculate a numerical value, e.g., "1", for a joint cartilage defect in the region of interest 400 in a sectional image, e.g., with identification number 7 and a numerical value, e.g., "2", for bone fractures in the region of interest 400 in another sectional image, e.g., with identification number 11, in which case, the numerical value 800 for the region of interest 400 may be determined to be "3" by summing the numerical values "1" and "2".

Alternatively, the controller 140 may perform a neural network operation on each of at least one sectional image determined to have a feature, calculate a numerical value for the region of interest 400 of each of the at least one sectional image based on information resulting from the neural network operation for each of the at least one sectional image, and determine the numerical value 800 for the region of interest 400 by summing the calculated numerical values.

The information resulting from the neural network operation may include information about numerical values for the region of interest in each of the at least one sectional image determined to have the feature.

The neural network refers to machine learning that embodies a neural structure capable of performing deep learning, and as weight and bias corresponding to elements of the neural network keep changing, learning confidence is improved.

Specifically, the X-ray imaging apparatus 100 may continue to receive sectional images having the feature and corresponding numerical values from the outside , and the controller 140 may keep on updating the weight, bias, and activation function included in the neural network based on the sectional images having the feature and the corresponding numerical values received from the outside, thereby improving the inference of the neural network. The X-ray imaging apparatus 100 may update the weight, bias, and activation function included in the neural network based on a captured sectional image and the corresponding numerical value.

The neural network may be stored in the storage 160 in the form of a computer program, and may include a CNN that generates a features map output by convolution of sectional images and enters the features map to the neural network, but it is not limited thereto and may be implemented with another deep learning algorithm including an RNN.

Furthermore, the controller 140 may control the display 150 to display at least one of the numerical value 800 for each region of interest 400 in one of a plurality of sectional images corresponding to a plurality of locations and a sum of the numerical values 850.

For example, the display 150 may display numerical values 810, 820, and 830 for the respective regions of interest 410, 420, and 430 in one of the plurality of sectional images, as shown in FIG. 9, under the control of the controller 140.

Furthermore, the display 150 may display the sum of numerical values 850 by summing the respective numerical values 810, 820, and 830, as shown in FIG. 9, under the control of controller 140.

This may enable the user to determine whether the subject P has a disease and the extent of the disease for each region of interest 400 by checking the numerical value 800 that scores the disease of the subject P.

Furthermore, when receiving a choice of one of regions of interest 400 from the user through the input device 1subject P, the controller 140 may control the display 150 to display at least one of a sectional image having the feature in the chosen region of interest and a calculation ground 910 for the numerical value for the chosen region of interest.

For example, referring to FIG. 10, when receiving a choice of the first region of interest 410 from among the regions of interest 400 from the user through the input device 130, the controller 140 may control the display 150 to display a sectional image, e.g., with the identification number 7, having the feature corresponding to the first region of interest 410.

The controller 140 may also control the display 150 to display the calculation ground 910 for the numerical value 810 for the first region of interest 410. For example, the calculation ground 910 may indicate that the numerical value "2" is derived from narrowing of joint space and that the numerical value "1" is derived from a joint cartilage defect.

This may enable the user to receive a particular sectional image from which the user is able to tell the feature without need to check every one of the plurality of sectional images, and to determine a disease of the subject P more easily based on the calculation ground for the numerical value.

Furthermore, the controller 140 may display indicators 640 and 650 to indicate features that become the basis of determination of the numerical value 810 on the sectional image in an embodiment of the disclosure, as shown in FIG. 10.

In some embodiments of the disclosure, the controller 140 may receive a modification to the displayed numerical value 800 through the input device 130. The user may not agree with the calculation ground 910 for the numerical value displayed on the display 150 and thus modify the determined numerical value 800 through the input device 130 based on his/her own determination.

In this case, the controller 140 may modify the numerical value 800 based on an input through the input device 130 and allocate it for the corresponding region of interest 400. This may increase confidence in the progress of a disease by comparison with a subsequent examination.

In this regard, the feature-based numerical value may be determined based on preset calculation criteria as shown in FIG. 11. For example, the preset calculation criteria may be set based on the modified stoke ankylosing spondylitis spine score (mSASSS). Accordingly, the feature-based numerical value may be determined when a sectional image of the spine of the subject P is obtained.

For example, based on the preset calculation criteria, as for joint space narrowing, numerical value "0" may be assigned for a normal range and numerical value "1" may be assigned for an occasion of partial narrowing, numerical value "2" may be assigned for an occasion when the joint space is reduced to less than 50% of normal joint space, numerical value "3" may be assigned for an occasion when the joint space is reduced to more than 50% of normal joint space, and numerical value "4" may be assigned for an occasion of adhesion of joint. For example, a numerical value may correspond to a degree, a class or a score of a condition of a disease corresponding to a feature.

Although FIG. 11 shows only information about the joint space narrowing and cartilage defect in the preset calculation criteria, the calculation criteria are not limited thereto but may allocate various numerical values based on degrees of each type of the feature, such as joint space narrowing, joint cartilage defect, foreign body in joint, bone fractures, etc.

For example, the information about correlations between features and numerical values may include information about types of feature set based on the preset calculation criteria as shown in FIG. 11 and numerical values corresponding to the extent of them.

Furthermore, as the weight, bias, and activation function included in the neural network for determining the numerical values are kept updated based on the numerical values determined for the feature based on the sectional image having the feature and the preset calculation criteria, the information about the numerical values included in the information resulting from the neural network operation may be determined based on the preset calculation criteria.

How the X-ray imaging apparatus 100 compares a sectional image of the subject P, for which a plurality of sectional images are obtained, obtained in the current examination with a sectional image obtained previously when there exists the sectional image obtained previously will now be described in detail.

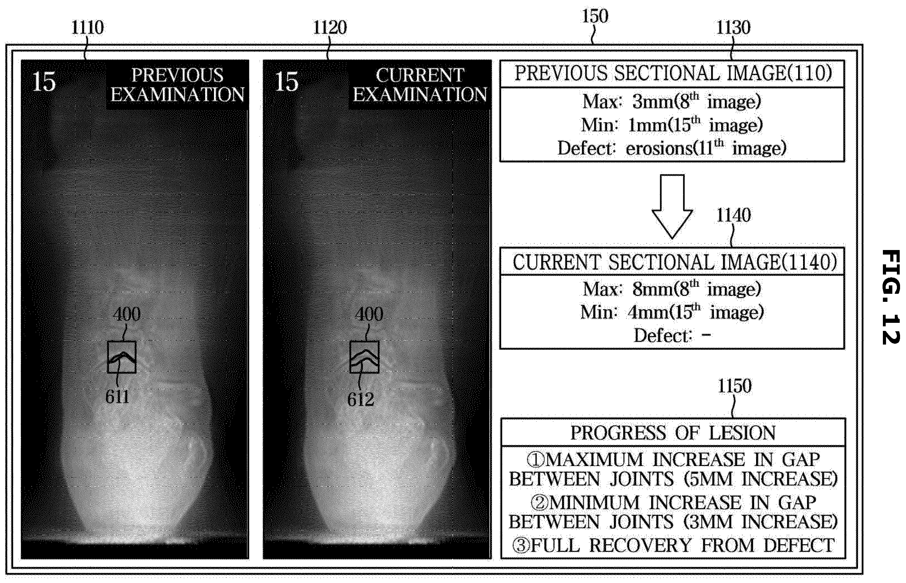

FIG. 12 shows an occasion when an X-ray imaging apparatus 100 displays a previous sectional image and a current sectional image for comparison, according to an embodiment of the disclosure.

Referring to FIG. 12, the controller 140 may determine whether there is a sectional image obtained previously for the subject P for which a plurality of sectional images are obtained, and when there is the previous sectional image of the subject P, may control the display 150 to display a current sectional image 1120 in the current examination along with a previous sectional image 1110.

Specifically, based on the patient information of the subject P, the controller 140 may determine whether there is a sectional image that corresponds to the patient information. In other words, on an occasion when an examination is currently performed on the subject P to obtain a plurality of sectional images, the controller 140 may determine whether there was a previous examination made on the subject P that obtained a sectional image.

In this case, the controller 140 may determine whether there is a previous sectional image of the subject P by comparing patient information assigned to a sectional image stored in the storage 160 with patient information for the subject P.

Alternatively, the controller 140 may determine whether there is a sectional image that corresponds to the patient information for the subject P among the sectional images stored, by communication with a PACS server that receives and stores sectional images obtained from the X-ray imaging apparatus 100 through a communicator (not shown).

In this regard, when there exist a plurality of previous sectional images that include the region of interest 400 corresponding to the current examination on the subject P and that were previously obtained, the controller 140 may control the display 150 to display at least one previous sectional image 1110 with an indicator 611 inserted to the region of interest 400 among the plurality of previous sectional images and at least one current sectional image 1120 with an indicator 612 inserted thereto in the current examination.

This may enable the user to receive a sectional image from the previous examination on the subject P as well as a sectional image from the current examination without need to search the sectional image from the previous examination, and to determine progress of the disease of the subject P more easily by comparing the sectional image from the current examination with the sectional image from the previous examination.

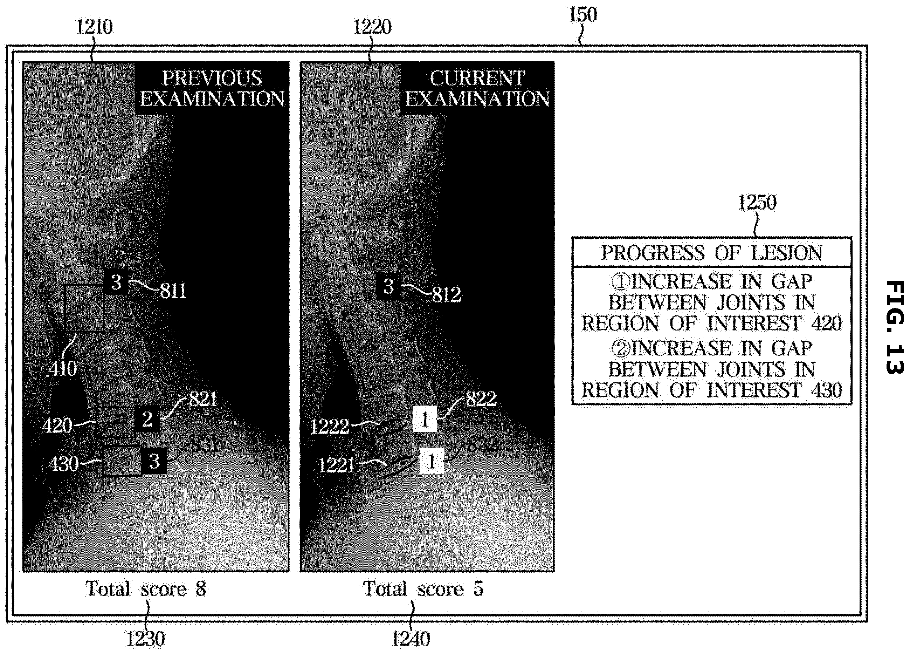

Furthermore, when there exist a plurality of previous sectional images that include the region of interest 400 corresponding to the current examination on the subject P and that were previously obtained, the controller 140 may control the display 150 to display at least one previous sectional image 1210 with numerical values 811, 821, and 831 for the regions of interest 410, 420, and 430 inserted thereto among the plurality of previous sectional images and at least one current sectional image 1220 with numerical values 812, 822, and 832 for the regions of interest 410, 420, and 430 inserted thereto in the current examination, as shown in FIG. 13.

Furthermore, the controller 140 may determine whether there is a change in the feature by comparing at least one sectional image from the current examination 1120 with at least one sectional image from the previous examination 1110.

Specifically, the controller 140 may determine at least one of whether there is a change in presence or absence of the feature, whether there is a change in thickness of a joint among the features, or whether there is a change in numerical value based on a state of the feature.

More specifically, the controller 140 may extract a feature from a previous sectional image based on an image process or neural network operation on the previous sectional image, and compare it with the feature in the sectional image from the current examination to determine at least one of whether there is a change in presence or absence of the feature, whether there is a change in thickness of a joint among the features, or whether there is a change in numerical value based on a state of the feature.

Subsequently, the controller 140 may control the display 150 to display information about the change determined in the feature. This may enable the user to determine progress of the disease of the subject P more easily.

For example, the controller 140 may control the display 150 to display information 1130 about feature types in the previous sectional image 1110 and identification numbers of sectional images corresponding to the feature types and information 1140 about feature types in the current sectional image 1120 and identification numbers of sectional images corresponding to the feature types at the same time in an embodiment of the disclosure, as shown in FIG. 12.

Furthermore, the controller 140 may control the display 150 to display information 1230 about a sum of numerical values in the previous sectional image 1210 and information 1240 about a sum of numerical values in the current sectional image 1220 at the same time in an embodiment of the disclosure, as shown in FIG. 13.

In addition, the controller 140 may control the display 150 to display information 1150 and 1250 about whether there is a change in the feature determined, as shown in FIGS. 12 and 13. The information 1150 and 1250 about whether there is a change in the feature may include information about a change in joint thickness, information about a change in presence or absence of a joint cartilage defect, a foreign body in joint or bone fractures, etc. That is, the information 1150 and 1250 about whether there is a change in the feature may correspond to the progress of a disease.

Moreover, the controller 140 may control the display 150 to display indicators 1221 and 1222 to indicate features that have changed, as shown in FIG. 13. Specifically, when the numerical values 822 and 832 are reduced as compared with the previous numerical values 821 and 831 due to an increase in gap between joints in the regions of interest 420 and 430, the controller 140 may control the display 150 to display the indicators 1221 and 1222 to indicate joints having the changed features.

A control method of the X-ray imaging apparatus 100 will now be described in accordance with an embodiment of the disclosure. For the control method of an X-ray imaging apparatus, the X-ray imaging apparatus 100 according to the previous embodiments may be used. Hence, what are described above with reference to FIGS. 1 to 13 may be equally applied in the following control method of the X-ray imaging apparatus 100 without being specifically mentioned.

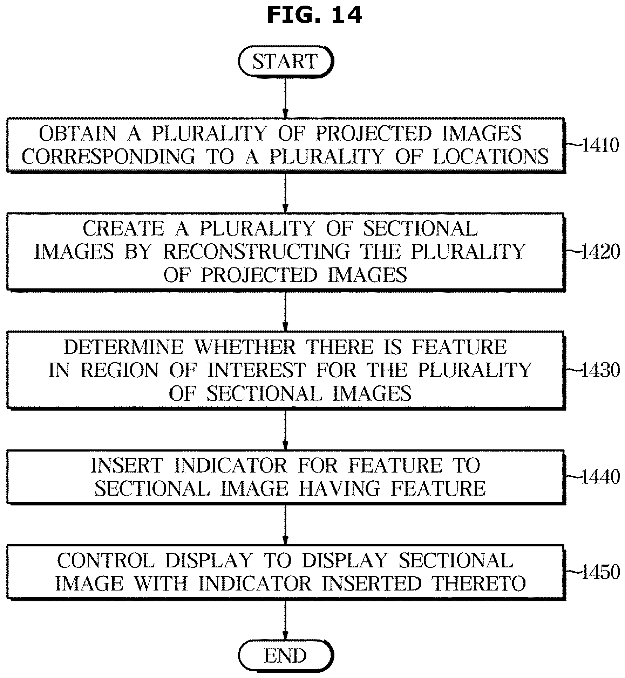

FIG. 14 is a flowchart of an instance of inserting an indicator to indicate a feature to a sectional image having the feature in a control method of an X-ray imaging apparatus 100, according to an embodiment of the disclosure.

Referring to FIG. 14, the X-ray imaging apparatus 100 obtains a plurality of projected images corresponding to a plurality of locations, in 1410.

Specifically, the controller 140 of the X-ray imaging apparatus 100 may receive an electric signal corresponding to an X-ray detected from the X-ray detector 120 and obtain a projected image based on the received electric signal.

As described above, the X-ray source 110 may irradiate X-rays to the subject P from the plurality of locations under the control of the controller 140. Accordingly, the controller 140 may create a projected image corresponding to each location of the X-ray source 110.

That is, the controller 140 may use the X-ray detected from the X-ray detector 120 to obtain a plurality of projected images corresponding to the plurality of locations from which the X-rays are irradiated from the X-ray source 110.

The X-ray imaging apparatus 100 creates a plurality of sectional images by reconstructing a plurality of projected images, in 1420. In this case, the controller 140 may allocate an identification number for each of the plurality of sectional images to distinguish one from another.

The X-ray imaging apparatus 100 determines whether there is a feature in the region of interest 400 for each of the plurality of sectional images, in 1430.

Specifically, the controller 140 may determine at least one sectional image having a preset feature in the region of interest 400 among the plurality of sectional images.

In other words, the controller 140 may determine whether there is the preset feature in the region of interest 400 for each of the obtained plurality of sectional images.

The region of interest 400 may be set up based on the user's input through the input device 130, as described above. Alternatively, the region of interest 400 may be set up by the controller 140 without the user's input. Specifically, the controller 140 may classify a particular portion (e.g., joints) that is likely to have a disease based on an image process, e.g., edge detection, morphological operation, or the like performed on the obtained sectional image, and establish an area including the classified particular portion as the region of interest. The particular portion may be preset in a design stage or set by the user through the input device 130.

The controller 140 may perform image processing on the region of interest of each of the plurality of sectional images to determine whether there is the preset feature in each of the plurality of sectional images.

Furthermore, the controller 140 may perform a neural network operation on each of the plurality of sectional images and determine whether there is the preset feature in each of the plurality of sectional images based on information resulting from the neural network operation for the sectional image.

How to determine whether there is the preset feature in each of the plurality of sectional images using the image processing or the neural network operation is already described above, so the detailed description thereof will not be repeated.

The X-ray imaging apparatus 100 inserts an indicator to indicate the feature to a sectional image having the feature, in 1440.

Specifically, the controller 140 of the X-ray imaging apparatus 100 may insert an indicator to indicate the detected feature to each of the at least one sectional image determined to have the preset feature.

Specifically, the controller 140 may insert the indicator to the sectional image to make the sectional image stand out among the plurality of sectional images, and the indicator may be inserted to a point corresponding to the feature.

The X-ray imaging apparatus 100 controls the display 150 to display the sectional image with the indicator inserted thereto, in 1450.

Specifically, the controller 140 of the X-ray imaging apparatus 100 may control the display 150 to display the at least one sectional image with the indicator inserted thereto. In this case, the controller 140 may control the display 150 to display both sectional images with or without the indicator inserted thereto, or control the display 150 to display only the sectional image with the indicator inserted thereto among the plurality of sectional images.

In the latter case, the controller 140 may control the display 150 to display only the sectional image with the indicator inserted thereto when receiving a preset input through the input device 130 in some embodiments of the disclosure.

For example, the controller 140 may control the display 150 to display at least one of the plurality of sectional images, and control the display 150 to display a sectional image in which the feature is found in a particular region of interest when receiving a choice of the particular region of interest from the user. In this case, the sectional image displayed on the display 150 may include the indicator to indicate the feature.

This may enable the user to receive a particular sectional image from which the user is able to tell the feature without need to check every one of the plurality of sectional images, and to determine more easily whether there is the feature through the indicator to indicate the feature.

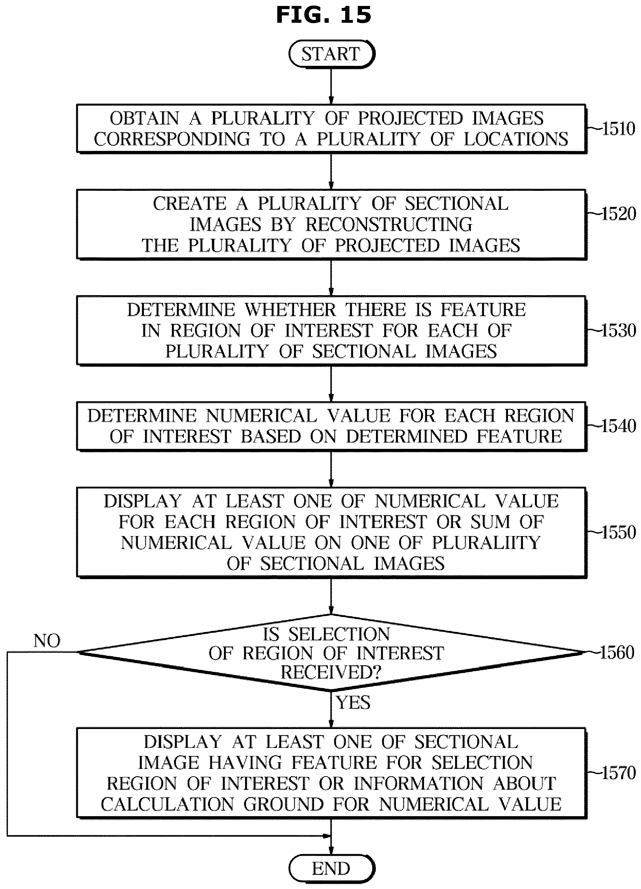

FIG. 15 is a flowchart of an instance of displaying a numerical value calculated based on a state of a feature in a control method of an X-ray imaging apparatus 100, according to an embodiment of the disclosure.

Referring to FIG. 15, as described above, the X-ray imaging apparatus 100 may obtain a plurality of projected images corresponding to a plurality of locations in 1510, create a plurality of sectional images by reconstructing the plurality of projected images in 1520, determine whether there is a feature in the region of interest 400 for each of the plurality of sectional images in 1530.

The X-ray imaging apparatus 100 determines the numerical value 800 for each region of interest 400 based on a state of the determined feature, in 1540.

Specifically, the controller 140 of the X-ray imaging apparatus 100 may determine the numerical value 800 that scores a disease based on a state of the determined feature.

The controller 140 may determine the numerical value 800 for the region of interest 400 based on a state of a feature in at least one sectional image determined to have the feature. The region of interest 400 may be set up in the singular or plural based on preferences.

In this regard, the controller 140 may determine the numerical value by using information about correlations between features and numerical values or based on information resulting from a neural network operation.

For example, the controller 140 may calculate numerical values for the region of interest 400 of each of at least one sectional image based on information about correlations between features and numerical values and the respective feature in the at least one sectional image, and determine the numerical value 800 corresponding to the region of interest 400 by summing the calculated numerical values.

Alternatively, the controller 140 may perform a neural network operation on each of at least one sectional image determined to have a feature, calculate a numerical value for the region of interest 400 of each of the at least one sectional image based on information resulting from the neural network operation for each of the at least one sectional image, and determine the numerical value 800 for the region of interest 400 by summing the calculated numerical values.

The X-ray imaging apparatus 100 displays at least one of the numerical value 800 for each region of interest in one of the plurality of sectional images and a sum of the respective numerical values 850, in 1550.

Furthermore, the controller 140 of the X-ray imaging apparatus 100 may control the display 150 to display at least one of numerical values for the respective regions of interest and a sum of the numerical values on one of the plurality of sectional images.

This may enable the user to determine whether the subject P has a disease and the extent of the disease for each region of interest by checking the numerical value that scores the disease of the subject P.

When receiving a choice of one of the regions of interest 400 from the user in 1560, the X-ray imaging apparatus 100 displays at least one of a sectional image having a feature corresponding to the chosen region of interest and information about the calculation ground 910 for the numerical value in 1570.

This may enable the user to receive a particular sectional image from which the user is able to tell the feature without need to check every one of the plurality of sectional images, and to determine a disease of the subject P more easily based on the calculation ground for the numerical value.

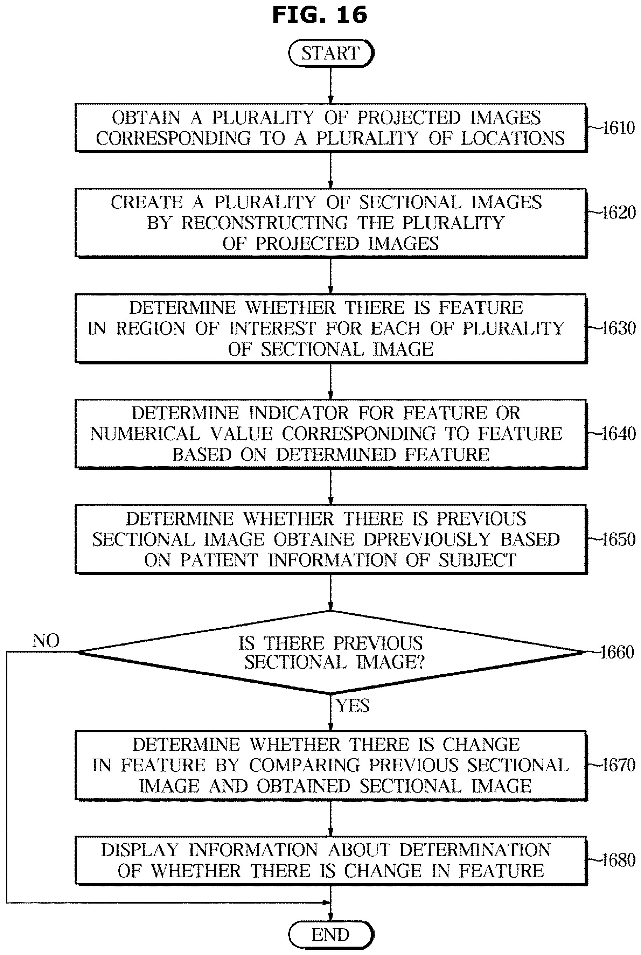

FIG. 16 is a flowchart of an instance of comparing a previously captured sectional image with a current sectional image in a control method of an X-ray imaging apparatus 100, according to an embodiment of the disclosure.

Referring to FIG. 16, as described above, the X-ray imaging apparatus 100 may obtain a plurality of projected images corresponding to a plurality of locations in 1610, create a plurality of sectional images by reconstructing the plurality of projected images in 1620, determine whether there is a feature in the region of interest 400 for each of the plurality of sectional images in 1630, and determine an indicator to indicate the feature or a numerical value corresponding to the feature based on the determined feature in 1640.

The X-ray imaging apparatus 100 determines whether there is a previous sectional image based on patient information of the subject P, in 1650.

Specifically, based on the patient information of the subject P, the controller 140 may determine whether there is a sectional image that corresponds to the patient information. In other words, on an occasion when an examination is currently performed on the subject P to obtain a plurality of sectional images, the controller 140 may determine whether there was a previous examination made on the subject P that obtained a sectional image.

In this case, the controller 140 may determine whether there is a previous sectional image of the subject P by comparing patient information assigned to a sectional image stored in the storage 160 with patient information for the subject P.

Alternatively, the controller 140 may determine whether there is a sectional image that corresponds to the patient information for the subject P among the sectional images stored, by communication with a PACS server that receives and stores sectional images obtained from the X-ray imaging apparatus 100 through a communicator (not shown).

When there is a previous sectional image in 1660, the X-ray imaging apparatus 100 determines whether there is a change in the feature by comparing the previous sectional image and the obtained sectional image, in 1670.

The controller 140 of the X-ray imaging apparatus 100 may determine whether there is a change in the feature by comparing at least one sectional image from the current examination 1120 with at least one sectional image from the previous examination 1110.

Specifically, the controller 140 may determine at least one of whether there is a change in presence or absence of the feature, whether there is a change in thickness of a joint among the features, or whether there is a change in numerical value based on a state of the feature.

More specifically, the controller 140 may extract a feature from a previous sectional image based on an image process or neural network operation on the previous sectional image, and compare it with the feature in the sectional image from the current examination to determine at least one of whether there is a change in presence or absence of the feature, whether there is a change in thickness of a joint among the features, or whether there is a change in numerical value based on a state of the feature.

Subsequently, the X-ray imaging apparatus 100 displays information about whether there is a change in feature determined, in 1680.

Specifically, the controller 140 may control the display 150 to display information about the determined change in the feature. This may enable the user to determine progress of the disease of the subject P more easily.

For example, the controller 140 may control the display 150 to display information 1130 about feature types in the previous sectional image 1110 and identification numbers of sectional images corresponding to the feature types and information 1140 about feature types in the current sectional image 1120 and identification numbers of sectional images corresponding to the feature types at the same time in an embodiment of the disclosure.

Furthermore, the controller 140 may control the display 150 to display information 1230 about a sum of numerical values in the previous sectional image 1210 and information 1240 about a sum of numerical values in the current sectional image 1220 at the same time in an embodiment of the disclosure.

In addition, the controller 140 may control the display 150 to display information 1150 and 1250 about whether there is a change in the feature determined, in an embodiment of the disclosure. The information 1150 and 1250 about whether there is a change in the feature may include information about a change in joint thickness, information about a change in presence or absence of a joint cartilage defect, a foreign body in joint or bone fractures, etc. That is, the information 1150 and 1250 about whether there is a change in the feature may correspond to the progress of a disease.

Meanwhile, the embodiments of the disclosure may be implemented in the form of recording media for storing instructions to be carried out by a computer. The instructions may be stored in the form of program codes, and when executed by a processor, may generate program modules to perform operation in the embodiments of the disclosure. The recording media may correspond to computer-readable recording media.

The computer-readable recording medium includes any type of recording medium having data stored thereon that may be thereafter read by a computer. For example, it may be a ROM, a RAM, a magnetic tape, a magnetic disk, a flash memory, an optical data storage device, etc.

According to an embodiment of the disclosure, an X-ray imaging apparatus and control method thereof is provided to allow the user to diagnose a disease more quickly by determining a sectional image including a feature corresponding to the disease among a plurality of sectional images corresponding to a plurality of locations and displaying an indicator to indicate the feature or a numerical value that scores the disease in the sectional image.

Several example embodiments have been described above, but a person of ordinary skill in the art will understand and appreciate that various modifications can be made without departing the scope of the disclosure. Thus, it will be apparent to those ordinary skilled in the art that the true scope of technical protection is only defined by the following claims.

## Claims

1. An X-ray imaging apparatus comprising:
an X-ray source irradiating X-rays to a subject from a plurality of locations;
an X-ray detector detecting X-rays irradiated from the plurality of locations and passing through the subject; and
a controller configured to obtain a plurality of projected images corresponding to the plurality of locations from the detected X-rays, create a plurality of sectional images by reconstructing the plurality of projected images, determine at least one of the plurality of sectional images having a preset feature in a region of interest, and insert an indicator to indicate the feature to the at least one sectional image.

2. The X-ray imaging apparatus of claim 1, further comprising: a display,
wherein the controller is configured to control the display to display the at least one sectional image to which the indicator is inserted.

3. The X-ray imaging apparatus of claim 1, wherein the preset feature comprises at least one type of a foreign body in a joint, a joint cartilage defect, bone fractures, a joint having maximum thickness, or a joint having minimum thickness, and
wherein the controller is configured to perform image processing on the region of interest in each of the plurality of sectional images by separating a joint image and a bone image included in the region of interest in each of the plurality of sectional images, and determine whether there is the preset feature in each of the plurality of sectional images.

4. The X-ray imaging apparatus of claim 3, wherein the controller is configured to determine at least one of whether there is a foreign body in the joint, whether there is a joint cartilage defect, or whether there is bone fracture in each of the plurality of sectional images based on image processing on the joint image and the bone image in each of the plurality of sectional images.

5. The X-ray imaging apparatus of claim 3, wherein the controller is configured to measure joint thickness in the region of interest in each of the plurality of sectional images based on the joint image and the bone image in each of the plurality of sectional images, determine at least one of a sectional image having maximum joint thickness or a sectional image having minimum joint thickness among the plurality of sectional images, and insert an indicator to indicate a joint to the determined sectional image.

6. The X-ray imaging apparatus of claim 3, wherein the controller is configured to perform a neural network operation on each of the plurality of sectional images, and determine whether there is the preset feature in each of the plurality of sectional images based on information resulting from the neural network operation for the sectional image.

7. The X-ray imaging apparatus of claim 2, wherein the controller is configured to control the display to match and display each type of the preset feature and an identification number of a sectional image corresponding to the type of the preset feature.

8. The X-ray imaging apparatus of claim 2, further comprising: an input device configured to receive an input of a user,
wherein the controller is configured to control the display to display one of the plurality of sectional images, receive a choice of at least one region of interest including a portion of interest in the sectional image from the user through the input device, and when a preset input is received from the user through the input device, control the display to display the at least one of the plurality of sectional images to which the indicator is inserted.

9. The X-ray imaging apparatus of claim 2, wherein the controller is configured to perform image processing on one of the plurality of sectional images to determine a joint included in the sectional image, and determine a region of interest to include the determined joint.

10. The X-ray imaging apparatus of claim 2, wherein the controller is configured to when there exist a plurality of previous sectional images of the subject including the region of interest and previously obtained, control the display to display at least one previous sectional image with an indicator inserted to the region of interest among the plurality of previous sectional images and at least one sectional image to which the indicator is inserted, determine whether there is a change in the feature by comparing the at least one sectional image with the at least one previous sectional image, and control the display to display information about the determination of whether there is a change in the feature.

11. An X-ray imaging apparatus comprising:
an X-ray source irradiating X-rays to a subject from a plurality of locations;
an X-ray detector detecting X-rays irradiated from the plurality of locations and passing through the subject; and
a controller configured to obtain a plurality of projected images corresponding to the plurality of locations from the detected X-rays, create a plurality of sectional images by reconstructing the plurality of projected images, determine at least one of the plurality of sectional images having a preset feature in a region of interest, and determine a numerical value corresponding to the region of interest based on a state of the feature in the at least one sectional image.

12. The X-ray imaging apparatus of claim 11, wherein the controller is configured to calculate a numerical value for the region of interest in each of the at least one sectional image based on information about correlations between features and numerical values and respective feature in the at least one sectional image, and determine a numerical value corresponding to the region of interest by summing the calculated numerical values.

13. The X-ray imaging apparatus of claim 11, wherein the controller is configured to perform a neural network operation on each of the at least one sectional image, calculate a numerical value for the region of interest of each of the at least one sectional image based on information resulting from the neural network operation for the sectional image, and determine a numerical value corresponding to the region of interest by summing the calculated numerical values.

14. The X-ray imaging apparatus of claim 11, further comprising: a display,
wherein the controller is configured to control the display to display at least one of numerical values of respective regions of interest in one of the plurality of sectional images or a sum of the numerical values.

15. A control method of an X-ray imaging apparatus comprising an X-ray source irradiating X-rays to a subject from a plurality of locations and an X-ray detector detecting X-rays irradiated from the plurality of locations and passing through the subject, the control method comprising
obtaining a plurality of projected images corresponding to the plurality of locations from the detected X-rays,
creating a plurality of sectional images by reconstructing the plurality of projected images,
determining at least one of the plurality of sectional images having a preset feature in a region of interest; and
inserting an indicator to indicate the feature to the at least one sectional image.
